# EUROPÄISCHE PATENTSCHRIFT

(11) **EP 2 340 805 B1**
(45) Veröffentlichungstag und Bekanntmachung des Hinweises auf die Patenterteilung: **04.01.2017**
(21) Anmeldenummer: 10190986.9
(22) Anmeldetag: 12.11.2010
(51) Int. Cl.: A61K 8/11, A61K 8/73, A61Q 19/00, A61Q 17/04, A61Q 19/08, A61K 8/92, A61K 8/97, A61K 8/67

(54) **Trägersystem zum Einschließen lipophiler Wirkstoffe und Öle in hoher Konzentration**
Holder system for enclosing lipophilic agents and oils in high concentrations
Système de support pour enfermer des matières actives et des huiles lipophiles en concentration élevée

(30) Priorität: 14.12.2009 DE 102009044891
(43) Veröffentlichungstag der Anmeldung: 06.07.2011
(73) Patentinhaber: Blume, Gabriele, 36396 Steinau an der Strasse (DE)
(72) Erfinder: Blume, Gabriele, 36396 Steinau an der Strasse (DE)
(74) Vertreter: Böck, Bernhard

(56) Entgegenhaltungen:
- EP-A1- 2 120 845
- EP-A2- 1 129 684
- WO-A1-2008/133482
- WO-A2-2009/080661

## Beschreibung

Die Erfindung betrifft die Verkapselung von schwer löslichen lipophilen Wirkstoffen, Ölen und/oder hydrophoben UV-Filtern in Form von Nanokapseln bestehend aus einem Lipidkern und einer kontinuierlichen Schale, die aus hydrophob modifiziertem Polysaccharid und einem membranbildenden Emulgator gebildet wird. Diese Kapseln liegen in einer wässerigen Dispersion vor, sodass die eingeschlossenen lipophilen Wirkstoffe in höherer Konzentration einfach in wasserbasierte Formulierungen eingearbeitet werden können, z.B. Sprühformulierungen, Gele oder Lotionen und O/W Emulsionen.

Die Verkapselung von lipophilen Wirkstoffen in Partikeln mit einer Größe im Submikrometerbereich ist seit vielen Jahren bekannt und wird insbesondere in der Kosmetik und Dermatologie häufig eingesetzt.

Aus dem europäischen Patent EP 1 342 471 (B1) sind Nanokapseln mit einer Größe von 200 bis 300 nm auf Basis von Polyolpolyestern bekannt, bei denen der Lipidkern u.a. aus Retinol/Retinolderivaten besteht. Durch die Verkapselung wird eine signifikante Stabilisierung des Vitamins erzielt. Die Herstellung dieser Kapseln ist großtechnisch sehr aufwendig (Abdampfen von Lösungsmitteln) und kostenintensiv.

Die EP 1 129 684 beschreibt eine Nanoemulsion (Nanokapseln von 150 nm), deren Lipidkern (Öltröpfchen) von einer Schale umschlossen wird, die aus einem amphiphilen Lipid und einem kationischen Polymer besteht. Hier ist hervorzuheben, dass das Gewichtsverhältnis von Ölmenge zu amphiphilem Lipid bei max. 6 liegt.

DE 60030835 offenbart eine transparente Nanoemulsion, bei der Öltröpfchen mit einer Größe von weniger als 150 nm ebenfalls von membranbildenden, amphiphilen Emulgatoren gegen die Wasserphase abgegrenzt werden. Hier liegt aber das Verhältnis von Ölanteil zu amphiphilem Lipid vorzugsweise im Bereich von 1,2 bis 6. Als Verdicker wird hier noch ein in Wasser dispergierbares Polymer zugesetzt, das sowohl hydrophile als auch hydrophobe Sequenzen mit einem Verhältnis von 10:1 bis 1000:1 enthält.

Auch liegen Beschreibungen vor, bei denen das hydrophob modifizierte Polysaccharid (Inutec SP 1) als Emulgator für Öl-in-Wasser-Emulsionen mit einem guten Hautgefühl eingesetzt wird. Henkel KgaA (DE 10 2005 038 069) setzt das hydrophob modifizierte Polysaccharid mit einem Copolymer ein, um eine emulgatorfreie Emulsion zu erhalten bzw. mit Cyclodextrinen und kleinen Emulgatoren (MG < 1000 g/mol bzw. < 1 %) (DE 10 2006 031 500), um ein gutes und sicheres Hautgefühl zu erhalten.

L'Oreal beschreibt ebenfalls in neueren Anmeldungen die Verwendung von hydrophob modifiziertem Inulin in Öl-in-Wasser-Emulsionen: es wird hierbei eine Emulsion beschrieben, bei der das Gewichtsverhältnis von Ölphase zu Inutec SP1 = 10 zu 0,1 beträgt; aber zur Stabilisierung noch ein pflanzlicher Gelbildner zugefügt werden muss (WO 2009/080659) oder ein hydrophiles Acrylpolymer wie z.B. AMPS (WO 2009/080661).

Die Firma ORAFTI (Hersteller des hydrophob modifizierten Inulin, Inutec SP1) gibt in ihrem Formulierungshandbuch an, dass stabile Öl-in-Wasser-Emulsionen bei einem Verhältnis von Öl zum Inutec SP1 von 10:1,2 entstehen.

Auch die Verkapselung von UV-Filtern ist vielfach beschrieben, zur Stabilisierung der Filter, insbesondere der Photostabilisierung, oder aber auch um Sonnenschutzmittel auf die Haut aufzubringen, ohne dass sie im direkten Kontakt mit dieser stehen.

DE 69208503 beschreibt Mikrokapseln mit einer Größe von 30 bis 80 µm bestehend aus gehärteter Gelantine und UV-Filtern, die nach Filtration und Trocknung in eine Sonnenschutzformulierung eingearbeitet werden können.

Die in DE 10 2007 035 567 beschriebenen Kapseln besitzen ebenfalls einen Lipidkern bestehend aus einem Emollient, in dem der hydrophobe schwerlösliche UV-Filter bis zu 40% gelöst wird, und einer den Kern umgebenden polymeren Hülle (Kieselgelschale).

Die Firma Merck vermarktet seit neuestem Mikrokapseln mit eingeschlossenem UV-Filter - Eusolex® UV-Pearls™ - welche den neuen Sonnenschutz darstellen. Mit der einzigartigen Technik der Mikroverkapselung werden organische Sonnenschutzfilter in Sol-Gel-Glas eingeschlossen (DE 60033279). Als erstes Produkt dieser Art steht verkapseltes Ethylhexyl Methoxycinnamate zur Verfügung. In diese Silica Mikrokapseln wurde ebenfalls 30% der Filter Octocrylene und Avobenzon (Verhältnis 3.5:1) verkapselt (Merck KGaA IFSCC Kongress Barcelona 2008).

Bisher noch nicht beschrieben ist die Kombination der Emulgatoren und auch die hohe Verkapselung an lipophilen Wirkstoffen im wässerigen Medium bzw. die hohe Verkapselung zweier UV-Filter mit dadurch erhöhter protektiver Wirkung im Sonnenschutz.

Es ergab sich somit die Aufgabe, Nanokapseln mit erhöhter Verkapselung an lipophilen Wirkstoffen bzw. UV-Filter bereitzustellen, die einfach zu produzieren sind.

Gelöst wird die Aufgabe durch die erfindungsgemäßen Nanokapseln, die die folgenden Bestandteile enthalten:
- einen flüssigen Lipidkern
- eine den Kern umgebende kontinuierliche Schale, gebildet aus mindestens einem hydrophob modifizierten Polysaccharid und mindestens einem membranbildenden Emulgator, wobei es sich bei dem Polysaccharid um Inulin handelt und wobei es sich bei dem Emulgator um GlycerylCitrate/Lactate/Linoleate/Oleate handelt.

Die Aufgabe wird ferner gelöst durch eine Zubereitung enthaltend Nanokapseln wie sie im Folgenden beschrieben werden.

Das hydrophob modifizierte Polysaccharid und/oder der membranbildende Emulgator sind vorzugsweise natürlichen, insbesondere pflanzlichen Ursprungs.

Die erfindungsgemäße Nanokapseln weisen eine negative Ladung auf und haben vorzugsweise eine Größe von 200 - 800 nm.

In einer bevorzugten Ausführungsform enthält die erfindungsgemäße Zubereitung noch einen Stabilisator (z.B. wasserlöslichen Alkohol oder Polyol) und ist damit als konservierungsmittelfrei anzusehen.

Die Nanokapseln enthaltende wässerige Zubereitung kann selbst als Kosmetikum / Pharmazeutikum eingesetzt werden, je nach enthaltenem Wirkstoff. Darüber hinaus lässt die Nanokapseln enthaltende wässerige Zubereitung sich in die Wasserphase einer weiteren Zubereitung wie eine Emulsion, Lotion, Creme oder unterschiedliche Formen von Gelen einarbeiten oder kann selbst verdünnt mit einem zugesetzten Verdicker als Sprühformulierung angewendet werden.

Die vorliegende Erfindung weist eine hohe Verkapselungseffizienz der lipophilen Stoffe auf. Die erfindungsgemäße Zubereitung enthält den flüssigen Lipidkern in einer Menge von 30 - 60 Gew.-%, bevorzugt von 40-55 Gew.-%, besonders bevorzugt von 50-55 Gew.-%, bezogen auf die Gesamtmenge der Zubereitung.

Weitere Vorteile der erfindungsgemäßen Zubereitung sind die hohe Stabilität der Komposition gegen Phasentrennung, ein gutes Hautgefühl und die leichte Verteilbarkeit auf der Haut.

Ein erfindungsgemäß besonders bevorzugtes Polysaccharid-Grundgerüst ist die lineare Polyfructose, insbesondere Inulin, gewonnen aus dem Chicoree.

Das Polysaccharid, insbesondere das Inulin, umfasst in einer bevorzugten Ausführungsform im Mittel von 2 bis 500, besonders bevorzugt von 25 bis 50 Monosaccharid-Einheiten.

Die hydrophobe Modifizierung wird erreicht durch Einbringen von vorzugsweise linearen C₁₀ bis C₁₈-Alkylgruppen. Die hydrophoben Gruppen sind über eine Alkyl-Etherbindung oder über eine Alkyl-Urethanbindung vorzugsweise an die Hydroxygruppen des Polysaccharids gebunden.

Die hier eingesetzten hydrophob modifizierten Polysaccharide sind grenzflächenaktiv.

Erfindungsgemäß besonders bevorzugt eingesetztes hydrophob modifiziertes Polysaccharid ist Polysaccharid-N-alkylurethane der Firma Orafti, vor allem Inulin Lauryl Carbamate (Inutec SP 1, Fa. Orafti), sowie Stearoyl Inulin (Lifidrem INST von Engelhard) und Palmitoyl Inulin (Rheopearl INS von Ciba).

Die hydrophob modifizierten Polysaccharide weisen sowohl hydrophile als auch lipophile Sequenzen auf in einem von Verhältnis < 10:1.

Die Menge an hydrophob modifizierten Polysacchariden in den erfindungsgemäßen Zubereitungen beträgt vorzugsweise zwischen 0,1 und 5,0 besonders bevorzugt zwischen 1 und 3,0 Gew.-%.

Neben dem hydrophob modifizierten Polysaccharid wird ein membranbildender (lamellare Strukturen bildender) Coemulgator eingesetzt, der als hydrophoben Teil langkettige Fettsäuren (z.B: Palmitin-, Stearin-, Öl- und / oder Linolsäure) enthält.

Diese Emulgatoren mit langer Alkylkette zeichnen sich durch eine hohe Hautverträglichkeit aus und können kosmetische und pharmazeutische Effekte erzielen (z.B. Erhöhung der Hautglätte, Reduzierung von Metalloproteasen, Reduzierung von Hautunreinheiten).

Besonders bevorzugt werden Glyceryl Citrate / Lactate / Linoleate / Oleate (Imwitor 375) bzw. Glyceryl Citrate / Lactate / Stearate von der Firma Sasol als Coemulgatoren zur Bildung der Kapselschale eingesetzt.

Die Menge an hydrophob modifizierten Polysacchariden (Inutec SP 1) in den erfindungsgemäßen Zusammensetzungen beträgt zwischen 1 und 3 Gew.-% und die des membranbildenden Coemulgators Glyceryl Citrate / Lactate / Linoleate / Oleate (Imwitor 375) zwischen 1 und 3 Gew.-%. Beide Komponenten bilden die Schale, die den flüssigen Lipidkern umgibt.

Der Lipidkern kann aus Ölen und Fettstoffen bestehen und in dieser Phase gelösten hydrophoben Wirkstoffen und / oder auch nur aus lipophilen Wirkstoffen z.B. nur UV-Filter.

Unter Fettstoffen sind vorzugsweise natürliche und synthetische kosmetische Öl-komponenten sowie natürliche und synthetische Wachse zu verstehen, die erfindungsgemäß bei Raumtemperatur flüssig sind. Diese Ölkomponenten sind physiologisch akzeptabel und hautfreundlich.

Bei dem Fettstoff kann es sich insbesondere um ein unpolares oder polares flüssiges Öl - das natürlich oder synthetisch sein kann - handeln. Die Ölkomponente kann insbesondere ausgewählt sein aus pflanzlichen Ölen, insbesondere Sonnenblumenöl, Olivenöl, Sojaöl, Rapsöl, Mandelöl, Orangenöl, Weizenkeimöl, Pfirsichkernöl, Sesamöl, Avocadoöl, Babassuöl, Hagebuttenöl, Nachtkerzenöl und die flüssigen Anteilen des Kokosöls. Geeignet sind aber auch andere Triglyceridöle, wie synthetische 6 Triglyceridöle, flüssige Paraffinöle aus synthetischen Kohlenwasserstoffen, sowie aus flüchtigen und nichtflüchtigen Siliconölen.

Weiterhin können eingesetzt werden die Di- und Trifettsäureester, insbesondere Trifettsäureester, von gesättigten und/oder ungesättigten linearen und/oder verzweigten Fettsäuren, insbesondere C₆-C₂₂ Fettsäuren, mit Glycerin, wie beispielsweise Triglyceride der Caprinsäure und/oder Caprylsäure wie (Neutralöl) bzw. Triglyceride aus konjugierter Linolsäure (CLA) wie Clarinol G-80 von LipidNutrition.

Als natürliche oder synthetische Wachse können erfindungsgemäß eingesetzt werden flüssige Pflanzenwachse wie Jojobawachs.

Erfindungsgemäß ist jede beliebige Kombination der oben genannten Fettstoffe einsetzbar.

Diese Öle können auch allein als kosmetisch/pharmazeutischer Rohstoff dienen.

In einer erfindungsgemäß ganz besonders bevorzugten Ausführungsform enthalten die Nanokapseln ein natürliches pflanzliches Öl, das nach seiner physiologischen Funktion ausgewählt wird: z.B. Aprikosenöl bzw. Olivenöl für die trockene Haut oder Nachtkerzenöl für die empfindliche Haut.

Die Öle und flüssigen Fettstoffe dienen auch als Lösungsmittel für hydrophobe Wirkstoffe wie Retinol in Sonnenblumenöl.

Die erfindungsgemäßen Nanokapseln enthalten mindestens ein hydrophob modifiziertes Polysaccharid, insbesondere Inulin Lauryl Carbamate, in einer Menge von 1-3 Gew.-%, mindestens einen membranbildenden Coemulgator in einer Menge von 1-3 Gew.-%, mindestens ein polares oder unpolares Öl in einer Menge von 0 bis 60 Gew.-% und / oder hydrophobe kosmetisch oder pharmazeutisch relevante Wirkstoffe.

Die erfindungsgemäße Zubereitung kann einen und / oder mehrere lipophile Wirkstoffe enthalten. Als solche Substanzen sind alle Stoffe anzusehen, die sich in einem Öl bei Raumtemperatur oder unter Wärme lösen lassen und kosmetisch und/oder pharmazeutisch relevante Effekte in oder auf der Haut ausüben. Bevorzugt sind diese Aktiva natürlichen Ursprungs, besonders bevorzugt pflanzlichen Ursprungs. Ein Auszug der kosmetisch und / oder dermatologisch wirksamen lipophilen Substanzen, die eingesetzt werden können, kann bestehen aus: Algen- und Pflanzenextraken (z.B. Phlorogine von Biotechmarine, ein Algenextrakt zur Behandlung von fettiger Haut oder z.B. Incromega V3 von Croda, ein Pflanzenextrakt von Echiomega); Anti-Cellulite wirksame Substanzen (z.B. CLA); Anti-Elastase und Anti-Collagenase wirksame Stoffe (z.B. ungesättigte Fettsäuren, wie Ölsäure oder EPA); antiinflammatorisch wirksame Stoffe (z.B. EPA = Eicosapentaensäure); Antioxidantien (z.B: Salbei Extrakt von Flavex, Liponsäure und deren Derivate); Ceramide (z.B. verschiedene Ceramide der Firma Cosmoferm); hautberuhigende und hautglättende Wirkstoffe (z.B. Bisabolol); Moisturiser (z.B. Glycerol Monoisostearate, Sucrose Polysoyate); Flavonoide (zu den Flavonoiden gehören Flavanole, Flavanone, Anthocyanidine, Flavone und Flavonole wie z.B. Sinensetin oder Polyphenole wie die im Grüntee oder Trauben); Phytosterole (wie β-Sitosterol aus Maisfaseröl); Radikalfänger (z.B. Ubichinol-Derivate wie Coenzym Q10); Saponine (z.B. vom Ginseng, Süßholzwurzel und Rosskastanie); sauerstoffbindende Substanzen (z.B. Perfluordekalin); sebumreduzierende Substanzen (z.B. 10-Hydroxydecansäure); Stoffe, die die Durchblutung und damit die Versorgung der Haut fördern (z.B. Nikotinsäureester); Terpene (kosmetisch und dermatologisch relevante Terpene sind aufgeführt in der Pharmazeutischen Zeitung Heft 22; 2006 von Sebastian Jäger u.a.); Vitamine (Retinol und Derivate, Vitamin E und Derivate wie Tocotrienole oder Carotine und Carotinoide wie Lycopene, Lutein oder Fucoxanthin, Vitamin D und Derivate).

Alle diese Wirkstoffe sollen die Hautalterung und / oder das Photoageing (durch UV- bzw. Umweltbelastung) verhindern bzw. inhibieren; die Synthese von dermalen und epidermalen Makromolekülen stimulieren bzw. deren Degradation verhindern; die Proliferation von Fibroblasten und Keratinozyten anregen und somit den Schutz und die Erhaltung einer gesunden Haut bewirken.

In einer weiteren bevorzugten Ausführungsform zum Sonnenschutz enthalten die erfindungsgemäßen Zubereitungen mindestens eine bzw. mehrere lipophile organische UV-Filtersubstanzen. Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z.B. Wärme, wieder abzugeben. Man unterscheidet UV-A Filter und UV-B Filter. Die UV-A und UV-B Filter können sowohl einzeln als auch in Mischungen eingesetzt werden. Der Einsatz von Filtermischungen ist erfindungsgemäß bevorzugt.

Unter den lipophilen UV-Filtersubstanzen, die erfindungsgemäß eingesetzt werden können, befinden sich zumeist Derivate aus der Familie der Zimtsäure; des Dibenzoylmethans, der Salicylsäure, des Camphers; der Triazine, welche in den folgenden Patenten beschrieben werden (US 4,367,390, EP 863145, EP 517104, EP 570838, EP 796851, EP 775698, EP 878469, EP 933376, EP 507691, EP 507692, EP 790243, EP 944624); des Benzophenons; der Diphenylacrylsäure, des Benzotriazols; der Benzalmalonsäure (im besonderen welche im Patent US 5,624,663 beschrieben sind); des Benzimidazols, des Imidazolines; der p-Aminobenzoesäure (PABA); der Benzoxazole (beschrieben in den Patenten EP 0832642, EP 1027883, EP 1300137 and DE 101 62 844); der 4,4 -Diarylbutadienkarbonsäure (beschrieben in DE 197 55 649, EP 916335, EP 1133980, EP 1133981 and EP-A-1008586), der Dimeren der Alkylstyrene (beschrieben in DE 198 55 649) und polymere Filter und Silikonfilter (beschrieben in WO 93/04665).

### Einige Beispiele für organische lipophile Filter:

Para-Aminobenzoesäure Derivate: Ethyl PABA, Ethyl Dihydroxypropyl PABA, Ethylhexyl Dimethyl PABA (Escalol 507 von ISP).
Salicylsäure Derivate: Homosalate (Eusolex HMS von Merck), Ethylhexyl Salicylate (Neo Heliopan OS von Symrise), TEA Salicylate (Neo Heliopan TS von Symrise).
Dibenzoylmethan Derivate: Butyl Methoxydibenzoylmethan (Eusolex 9020 von Merck) und Isopropyl Dibenzoylmethan.
Zimtsäure Derivate: Ethylhexyl Methoxycinnamate (Parsol MCX von Roche Vitamins), Isopropyl Methoxycinnamate, Isoamyl Methoxycinnamate (Neo Heliopan E 1000 von Symrise), Cinoxate, Diisopropyl Methylcinnamate.
Diphenylacrylsäure Derivate: Octocrylene (Eusolex OCR von Merck), Etocrylene (Uvinul N35 von BASF).
Benzophenone Derivate: Benzophenone-1 (Uvinul 400 von BASF), Benzophenone-2 (Uvinul D50 von BASF), Benzophenone-3 oder Oxybenzone (Uvinul M40 von BASF), Benzophenone-6, (Helisorb 11 von Norquay), Benzophenone-8, Spectra-Sorb UV-24 von American Cyanamid, Benzophenone-9 (Uvinul DS-49 Benzophenone von BASF).
Benzylidene Camphor Derivate: 3-Benzylidene Camphor (Mexoryl SD von Chimex), 4-Methylbenzylidene Camphor (Eusolex 6300 von Merck).
Triazin Derivate: Bis-Ethylhexyloxyphenol Methoxyphenyl Triazine (Tinosorb S von Ciba Geigy), Ethylhexyl triazone (Uvinul T150 von BASF), Diethylhexyl Butamido Triazone (Uvasorb Heb von Sigma 3V) 2,4,6-tris(dineopentyl 4'-aminobenzalmalonate)s-triazine, 2,4,6-tris(diisobutyl 4'-aminobenzalmalonate)s-triazine.
Benzotriazol Derivate: Drometrizole Trisiloxane (Silatrizole von Rhodia Chimie).
Anthranilsäure Derivate: Menthyl anthranilate (Neo Heliopan MA von Symrise).
Imidazolin Derivate: 25 Ethylhexyl Dimethoxybenzylidene Dioxoimidazoline Propionate.
Benzalmalonate Derivate: Dineopentyl 4'-methoxybenzalmalonate.
Polyorganosiloxane mit Benzalmalonat FunKtion: Polysilicone-15 (Parsol SLX von Roche Vitamins).

Einige der öllöslichen UV-Filter können selbst als Lösungsmittel oder Lösungsvermittler für andere UV-Filter dienen. So lassen sich beispielsweise Lösungen des UV-A Filters 4-tert.-Butyl- 4'methoxybenzoylmethane (Avobenzone oder Eusolex 9020 von Merck) in verschiedenen UV-B Filtern herstellen. Die erfindungsgemäßen Zubereitungen enthalten daher in einer weiteren bevorzugten Ausführungsform 4-tert.-Butyl- 4'methoxybenzoylmethane (Avobenzone oder Eusolex 9020 von Merck) in Kombination mit mindestens einem UV-B Filter, ausgewählt aus Octrocrylene (Eusolex OCR von Merck) oder Ethylhexyl Dimethyl PABA (Eusolex 6007 von Merck) oder Homosalate (Eusolex HMS von Merck) oder Ethylhexyltriazon (Eusolex OS von Merck).

In der erfindungsgemäß beschriebenen bevorzugten Kombination liegt das Gewichtsverhältnis von UV-B-Filter (Octocrylene) zu UV-A-Filter (Avobenzone) zwischen 5:1 und 10:1, bevorzugt zwischen 3:1 und 4:1.

Die bevorzugten erfindungsgemäßen Zubereitungen zum Sonnenschutz sind dadurch gekennzeichnet, dass sie mindestens eine organische UV-Filtersubstanz in einer Gesamtmenge von 0,1 - 40 Gew.-%, bevorzugt 10 - 40 Gew.-%, jeweils bezogen auf die gesamte Zubereitung, enthalten. Besonders vorteilhaft ist die Kombination eines UV-A- und UV-B-Filters in einer Gesamtmenge von 30 - 60 Gew.-%, bevorzugt von 40 - 55 Gew.-%, besonders bevorzugt von 50 - 55 Gew.-%, bezogen auf die gesamte Zubereitung.

Die erfindungsgemäße kosmetische oder pharmazeutische Zubereitung für den Sonnenschutz kann auch weitere Bestandteile als die zuvor genannten enthalten. In einer bevorzugten Ausführungsform enthält sie mindestens eine der im Folgenden aufgezählten Substanzen. Sie kann auch jede beliebige Kombination der bei den Wirkstoffen und Ölen aufgezählten Bestandteile enthalten.

In einer erfindungsgemäß bevorzugten Ausführungsform liegt das Gewichtsverhältnis von flüssigem Lipidkern zur Schale im Bereich von 8:1 bis 16:1.

Die erfindungsgemäßen Zubereitungen können weiterhin wenigstens einen Stabilisator, der vorzugsweise ein wasserlöslicher Alkohol ist, enthalten. Der Stabilisator / Solvent wird vorzugsweise in einer Konzentration von 10 - 20 Gew.-%, bezogen auf die gesamte Zubereitung, eingesetzt. Geeignet sind je nach Darreichungsform einwertige Alkohole wie z.B. Ethanol, Propanol oder Isopropanol. Weiterhin geeignet sind wasserlösliche Polyole. Hierzu zählen wasserlösliche Diole, Triole und höherwertige Alkohole. Unter den Diolen eignen sich C₂ bis C₁₀- Diole, insbesondere 1,2-Propylenglycol, 1,2-Butylenglycol, 1,3-Butylenglycol und 1,4-Butylenglycol, 1,2-Pentadiol, 1,6-Hexandiol und 1,2-Octandiol. Weiterhin bevorzugt geeignet sind Gly-cerin und insbesondere Diglycerin und Triglycerin, 1,2,6-Hexantriol sowie die Dipropylenglycole. Für die Herstellung der erfindungsgemäßen Nanokapseln werden bevorzugt Ethanol oder 1,2-Pentandiol (Hydrolite 5 von Symrise) eingesetzt, in Konzentrationen von 10 bis 30 Gew.-%, besonders bevorzugt von 15 - 20 Gew.-%, bezogen auf die gesamte Formulierung, sodass somit auf eine andere Konservierung (klassische Konservierungsmittel) verzichtet werden kann. Die erfindungsgemäße Zubereitung kann deshalb als konservierungsmittelfrei deklariert werden.

Die hier beschriebene Erfindung beinhaltet ebenfalls den Gebrauch der Nanokapseln in kosmetisch und dermatologisch interessanten wässerigen Formulierungen (Sprühformulierung, unterschiedlichste Gele, Lotionen und Cremes) sowie deren Anwendung zur Behandlung, zum Schutz und Pflege der Haut, Haare, Nägel und Lippen.

Im Folgenden soll das Verfahren zur Herstellung einer erfindungsgemäßen Zubereitung anhand von einigen Ausführungsbeispielen und Abbildungen, die nicht als einschränkend zu verstehen sind, demonstriert werden.
- Fig. 1: UV-Nanokapseln als sprühbare wässrige Sonnenschutzformulierung wiedergegeben in einer 600-fachen Vergrößerung;
- Fig. 2: freier Filter verdickt in Wasser wiedergegeben in einer 600-fachen Vergrößerung.

### Beispiel 1

### UV-Filter Nanokapsel für den Sonnenschutz

a) 11,11 g Eusolex 9020 (UV-A Filter von Merck; Avobenzone) wird unter Hitze (70°C) in 38,89 g Eusolex OCR (UV-B Filter von Merck, Octocrylen) gelöst bis eine klare gelbe Flüssigkeit entsteht.
b) 2 g Inutec SP1 (hydrophob modifiziertes Inulin von Orafti) und 1,5 g Imwitor 375 (Coemulgator von Sasol; Glyceryl Citrate / Lactate / Linoleate / Oleate) werden in 16 g Ethanol dispergiert. Danach wird unter Rühren 30,5 g Wasser zugegeben. Unter ständigem Rühren und Homogenisieren wird die Lipidphase (UV-Filter Lösung) dem Wasser-Ethanol-Emulgator-Gemisch zugefügt. Diese Emulsion wird anschließend 5 Minuten bei 200 bar hochdruckhomogenisiert.

Die entstandenen UV-Nanokapseln weisen eine Teilchengröße zwischen 700 und 800 nm auf und besitzen eine negative Oberflächenladung.

Man erhält eine sprühbare, wässerige Sonnenschutzformulierung, in dem man die enstandenen UV-Nanokapseln anschließend in Wasser verdünnt (10 g UV-Filterkapseln + 33 g Wasser) und zu der Wasserphase unter starkem Homogenisieren eine Verdickersuspension (0,25 g Ketrol; Xanthan Gum von Kelco dispergiert in 6,5 g Ethanol) hinzugefügt. Diese sprühbare Sonnenschutzfomulierung zerläuft nicht auf der Haut und ergibt einen homogenen Sonnenschutz, welches wie folgt nachgewiesen wurde.

### Bestimmung des "Radical Skin Protection Factor (RSF)"

Wird die Haut der Sonnenstrahlung, insbesondere der UV-A und UV-B-Strahlung ausgesetzt, kommt es zu Schädigungen in der Haut. UV-A Strahlung induziert die Bildung freier Radikale, im speziellen reaktive Sauerstoffradikale (ROS), die dann mit anderen Bestandteilen der Haut in Wechselwirkung treten und zur Hautalterung führen. Die UV-B Strahlung interagiert direkt mit der DNA und kann schlimmstenfalls zum Hautkrebs führen.

Die ESR (Elektron Spin Resonanz Spektroskopie) stellt eine Methode dar, freie Radikale in der Haut quantitativ zu bestimmen (siehe Web-Seite von Gematria Test Lab). Hierbei wird die Epidermis einer Hautbiopsie mit einem Marker getränkt, der später mit den entstehenden freien Radikalen reagiert und das daraus entstandene Adukt wird dann spektroskopisch erfasst. Die zu untersuchenden Formulierungen werden auf die Haut appliziert (2 mg / cm²); dann wird die Haut mittels eines Sonnensimulators definiert bestrahlt. Der RSF für die UV-Nanokapseln (1:5 verdickt mit Xantham Gum) ergibt einen Wert von 7,4 +/- 0,4; das bedeutet eine Reduzierung der Bildung freier Radikale um 86%. Die geringe Standardabweichung weist auf eine homogene Verteilung der Kapseln auf der Haut hin. Werden die Filter frei / unverkapselt in eine Sprühformulierung (1:5 verdickt mit Xantham Gum) eingearbeitet, so ergibt sich ein RSF von 4,9 +/- 0,9. Hierbei ist der Sonnenschutz geringer und weist eine inhomogene Verteilung auf.

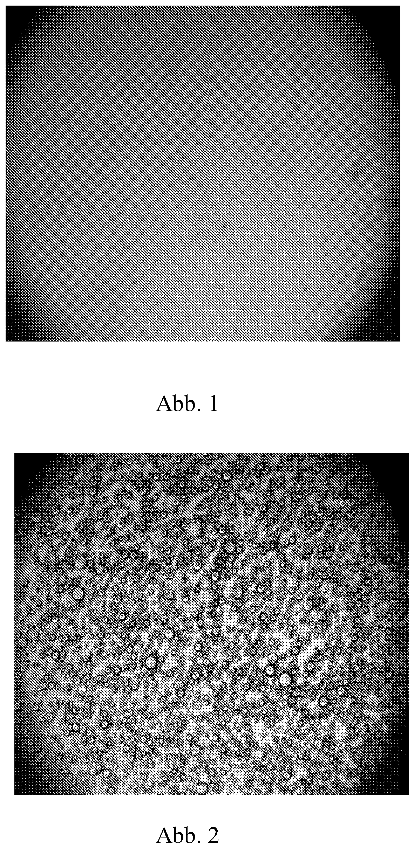

Die verbesserte Verteilung der verkapselten UV-Filter gegenüber den unverkapselten UV Filtern wird in den Abbildungen 1 und 2 verdeutlicht. Abb. 1 zeigt UV-Nanokapseln, als sprühbare wässrige Sonnenschutzformulierung (Formulierung siehe oben). Abb. 2 stellt freie Filter, die in Wasser verdickt werden, dar. Beide Abbildungen werden in einer 600-fachen Vergrößerung wiedergegeben. Die UV-Nanokapseln bilden eine homogene Dispersion und lichtmikroskopisch nicht sichtbare Teilchen (< 1 µm), dagegen weisen die freien Filter eine große Tröpfchengröße und eine sehr inhomogene Verteilung auf.

Auch zeigt sich schon nach kurzer Zeit eine Phasentrennung bei der Formulierung mit den freien Filtern, d.h. ein Absetzen der Filter im Wasser und ein Auskristallisieren dieser, so dass eine solche Formulierung nicht vermarktungsfähig ist.

### Beispiel 2

### EPA-Salbei Nanokapseln zum Hautschutz

a) 0,5 g Salbei Antioxidans Extrakt (von der Firma Flavex) und 2,5 g IncromegaV3 (pflanzliches EPA von Croda) werden unter Wärme (30°C) in 47,0 g Sonnenblumenöl gelöst.
b) 2 g Inutec SP1 (hydrophob modifiziertes Inulin von Orafti) und 1,5 g Imwitor 375 (Coemulgator von Sasol; Glyceryl Citrate / Lactate / Linoleate / Oleate) werden in 20 g 1,2-Pentylenglycol dispergiert. Danach wird unter Rühren 26,5 g Wasser zugegeben.

Unter ständigem Rühren und Homogenisieren wird die Lipidphase (Öl-Lösung) dem Wasser-Ethanol-Emulgator-Gemisch zugefügt. Diese Emulsion wird anschließend 5 Minuten bei 200 bar hochdruckhomogenisiert.

Die entstandenen Hautschutz-Nanokapseln weisen eine Teilchengröße von 220 nm auf und besitzen eine negative Oberflächenladung.

### Beispiel 3

### Retinol Nanokapseln gegen Hautalterung

a) 50 g Retinol 10S (10% Retinol in Sonnenblumenöl gelöst von BASF)
b) 2 g Inutec SP1 (hydrophob modifiziertes Inulin von Orafti) und 1,75 g Imwitor 375 (Coemulgator von Sasol; Glyceryl Citrate / Lactate / Linoleate / Oleate) werden in 20g 1,2-Pentylenglycol dispergiert. Danach wird unter Rühren 26,25 g Wasser zugegeben.

Unter ständigem Rühren und Homogenisieren wird die Lipidphase (Retinol- Lösung) dem Wasser-Ethanol-Emulgator-Gemisch zugefügt. Diese Emulsion wird anschließend 5 Minuten bei 200 bar hochdruckhomogenisiert.

Die entstandenen Retinol-Nanokapseln mit einem Wirkstoffgehalt von 5% reinem Retinol weisen eine Teilchengröße von 280 nm auf und besitzen eine negative Oberflächenladung.

## Patentansprüche

1. Nanokapseln, enthaltend mindestens die folgende Bestandteile:
- einen flüssigen Lipidkern
- eine den Kern umgebende kontinuierliche Schale, gebildet aus mindestens einem hydrophob modifizierten Polysaccharid und mindestens einem membranbildenden Emulgator, wobei es sich bei dem Polysaccharid um Inulin handelt und wobei es sich bei dem Emulgator um GlycerylCitrate/Lactate/Linoleate/Oleate handelt.

2. Nanokapseln nach Anspruch 1, **dadurch gekennzeichnet, dass** sie eine Größe von 200 bis 800 nm aufweisen.

3. Nanokapseln nach einem der Ansprüche 1 bis 2, **dadurch gekennzeichnet, dass** es sich bei dem hydrophob modifizierten Polysaccharid bevorzugt um Inulin Lauryl Carbamate handelt.

4. Nanokapseln nach einem der Ansprüche 1 bis 3, **dadurch gekennzeichnet, dass** das Massenverhältnis von flüssigem Lipidkern zur Schale im Bereich von 8:1 bis 16:1 liegt.

5. Wässrige Zubereitung, enthaltend Nanokapsein nach einem der Ansprüche 1 bis 4, wobei die Menge an Inulin 1-3 Gew. %, die Menge an GlycerylCitrate/Lactate/Linoleate/Oleate 1-3 Gew. % und die Menge an flüssigem Lipidkern 40 - 60 Gew. % bezogen auf die Gesamtmenge der Zubereitung, beträgt.

6. Zubereitung nach Anspruch 5, **dadurch gekennzeichnet, dass** sie weiterhin mindestens einen Stabilisator in Form von Alkoholen oder Polyolen enthält.

7. Zubereitung nach einem der Ansprüche 5 bis 6 zum Schutz der Haut des menschlichen oder tierischen Körpers.

8. Verwendung der Zubereitung nach einem der Ansprüche 5 bis 7 für einen kosmetischen und/oder nicht- therapeutischen, dermatologischen Zweck.

## Claims

1. Nanocapsules, comprising at least the following components:
- a liquid lipid core
- a continuous shell surrounding the core, said shell formed by at least one hydrophobically modified polysaccharide and at least one membrane-forming emulsifier, wherein the polysaccharide is inulin and wherein the emulsifier is glyceryl citrate/lactate/linoleate/oleate.

2. The nanocapsules according to claim 1, **characterized in that** they have a size of 200 to 800 nm.

3. The nanocapsules according to any one of the claims 1 to 2,
**characterized in that** the hydrophobically modified polysaccharide is preferably inulin lauryl carbamate.

4. The nanocapsules according to any one of the claims 1 to 3,
**characterized in that** the mass ratio of the liquid lipid core to the shell is in the range of 8:1 to 16:1.

5. An aqueous preparation, comprising nanocapsules according to any one of the claims 1 to 4, wherein the amount of inulin is 1 to 3% by weight, the amount of glyceryl citrate/lactate/linoleate/oleate is 1 to 3% by weight and the amount of liquid lipid core is 40 to 60% by weight, which is based on the total weight of the preparation.

6. The preparation according to claim 5, **characterized in that** it further comprises at least one stabilizer in the form of alcohols or polyols.

7. The preparation according to any one of the claims 5 to 6 for protecting the skin of the human or animal body.

8. Use of the preparation according to any one of claims 5 to 7 for a cosmetic and/or non-therapeutic, dermatologic purpose.

## Revendications

1. Nanocapsules comprenant au moins les composants suivants :
- un noyau lipide liquide
- une enveloppe continue entourant le noyau, formée par au moins un polysaccharide modifié dans une manière hydrophobe et au moins un émulsifiant formant membrane, le polysaccaride étant une inuline et l'émulsifiant étant un citrate/lactate/linoléate/oléate de glycéryle.

2. Nanocapsules selon la revendication 1, **caractérisé en ce qu**'elles ont une taille de 200 à 800 nm.

3. Nanocapsules selon l'une quelconque des revendications 1 à 2,
**caractérisé en ce que** le polysaccaride modifié dans une manière hydrophobe est en préférence de l'inuline lauryle carbamate.

4. Nanocapsules selon l'une quelconque des revendications 1 à 3,
**caractérisé en ce que** le rapport à masse du noyau lipide liquide à l'enveloppe est dans une gamme de 8 : 1 à 16 : 1.

5. Préparation aqueuse, comprenant des nanocapsules selon l'une quelconque des revendications 1 à 4, dans laquelle la quantité de l'inuline est 1 à 3 % en poids, la quantité du citrate/lactate/linoléate/oléate de glycéryle est 1 à 3 % en poids et la quantité du noyau lipide liquide est 40 à 60 % en poids, par rapport à la quantité totale de la préparation.

6. Préparation selon la revendication 5, **caractérisé en ce qu**'elle comprend en outre un stabilisateur en forme d'alcools ou de polyols.

7. Préparation selon l'une quelconque des revendications 5 à 6 pour la protection de la peau du corps humain ou animal.

8. Utilisation de la préparation selon l'une quelconque des revendications 5 à 7 dans un but dermatologique cosmétique et/ou non thérapeutique.
